# EUROPEAN PATENT APPLICATION

(11) **EP 1 903 116 A1**
(43) Date of publication of application: **26.03.2008**
(21) Application number: 06018328.2
(22) Date of filing: 01.09.2006
(51) Int. Cl.: C12Q 1/68

(54) **Supplementation of sequence based typing of methicillin-resistant S. aureus (MRSA) by PCR-based grouping of SCCmec-elements; sequencing directly from the multiplex-PCR**

(71) Applicant: Bundesrepublik Deutschland vertr. d. d. Bundes- ministerium für Gesundheit und Soziale Sicherung letztvertr. d.d. Präs. d. Robert Koch Instituts, 13353 Berlin (DE)
(72) Inventor: Cuny, Christa, 38855 Silstedt (DE); Witte, Wolfgang, 38875 Elend (DE)
(74) Representative: Krauss, Jan

(57) **Abstract**

The present invention relates to a *spa*-sequence based typing of MRSA, which is performed directly on the sample for the multiplex-PCR used for identifying the staphylococcal cassette chromosome mec (SCCmec)-groups. In particular, the method for typing strains or lineages of MRSA, comprises the steps of a) providing a DNA sample comprising DNA of an MRSA strain to be analysed, b) performing a multiplex DNA amplification of said DNA in said sample, wherein at least one sequence of an SCC*mec*-element and an X-region of a *spa-*gene are co-amplified, in order to produce a master amplification-mix, c) analysing the amplified SCCmec-element(s) and the X-region(s) of the spa-gene directly from said master amplification-mix, and d) typing said strains or lineages of MRSA based on said analysis.

## Description

The present invention relates to a *spa*-sequence based typing of MRSA, which is performed directly on the sample for the multiplex-PCR used for identifying the staphylococcal cassette chromosome mec (SCC*mec*)-groups*.* In particular, the method for typing strains or lineages of MRSA, comprises the steps of a) providing a DNA sample comprising DNA of an MRSA strain to be analysed, b) performing a multiplex DNA amplification of said DNA in said sample, wherein at least one sequence of an SCC*mec*-element and an X-region of a *spa*-gene are co-amplified, in order to produce a master amplification-mix, c) analysing the amplified SCC*mec*-element(s) and the X-region(s) of the *spa*-gene directly from said master amplification-mix, and d) typing said strains or lineages of MRSA based on said analysis.

The molecular typing of antibiotic-resistant bacterial pathogens in particular in hospital acquired infections is an important prerequisite for an effective and targeted use of infection control measures in hospitals in order to avoid a further dissemination. In doing so, it is necessary to both identify the base genome as well as transferable elements that encode for antibiotic-resistance conferring genes.

In methicillin-resistant *S. aureus* (MRSA), the beta-lactam resistance gene mecA is carried by a family of mobile genetic elements, designated staphylococcal cassette chromosome *mec* (SCC*mec*). Furthermore, genes designated as cassette chromosome recombinase genes (*ccr*) encoding polypeptides having a partial homology to recombinases of the invertase/resolvase family have been identified that are also located on SCC*mec* elements. The *ccr* were found to catalyze excision of the SCC*mec* from the methicillin-resistant *S. aureus* chromosome and site-specific as well as orientation-specific integration of the SCC*mec* into the *S. aureus* chromosome when introduced into the cells as a recombinant multicopy plasmid.

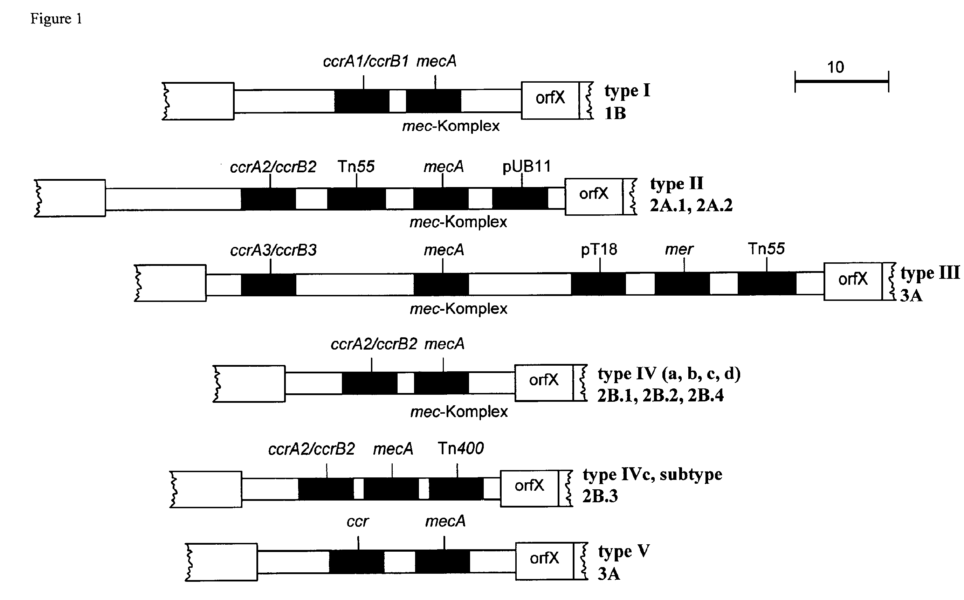

Until today, five basic types of SCC-elements (SCC type I to V) have been identified (see Figure 1). They all share two determinants for a recombinase (important for integration in or excision from the bacterial chromosome) as well as the more or less complete mec-regulon (*mec*A*, mec*R for response-regulator, and *mec*I for repressor). In addition, also transposons Tn554 (site-specific, contains *erm*A for macrolide-lincosamidin-resistance) as well as resistance-plasmids, such as pUB110, and pT181 (*tet*K*,* tetracycline resistance) can be integrated into SCC*mec*-elements. Additional DNA-sequences potentially encode for proteins having yet unknown function or which are carried over as evolutionary "ballast". Of particular interest is the gene *pls* that counteracts the mechanisms of the internalisation of *S*. *aureus* (Ito T, Katayama Y, Asada K, Mori N, Tsutsumimoto K, Tiensasitorn C, Hiramatsu K. Structural comparison of three types of staphylococcal cassette chromosome mec integrated in the chromosome in methicillin-resistant Staphylococcus aureus. Antimicrob Agents Chemother. 2001 May;45(5):1323-36. Katayama Y, Ito T, Hiramatsu K. A new class of genetic element, staphylococcus cassette chromosome mec, encodes methicillin resistance in Staphylococcus aureus. Antimicrob Agents Chemother. 2000 Jun;44(6):1549-55. Ito T, Ma XX, Takeuchi F, Okuma K, Yuzawa H, Hiramatsu K. Novel type V staphylococcal cassette chromosome mec driven by a novel cassette chromosome recombinase, ccrC. Antimicrob Agents Chemother. 2004 Jul;48(7):2637-51. Ito T, Katayama Y, Asada K, Mori N, Tsutsumimoto K, Tiensasitorn C, Hiramatsu K. Structural comparison of three types of staphylococcal cassette chromosome mec integrated in the chromosome in methicillin-resistant Staphylococcus aureus. Antimicrob Agents Chemother. 2001 May;45(5):1323-36. Chongtrakool P, Ito T, Ma XX, Kondo Y, Trakulsomboon S, Tiensasitorn C, Jamklang M, Chavalit T, Song JH, Hiramatsu K. Staphylococcal cassette chromosome mec (SCCmec) typing of methicillin-resistant Staphylococcus aureus strains isolated in 11 Asian countries: a proposal for a new nomenclature for SCCmec elements. Antimicrob Agents Chemother. 2006;50:1001-12).

Figure 1 shows an overview of the structures of yet well-characterised SCCmec elements. Their differentiation is of essential importance for the follow-up of the evolution of very particular epidemic MRSA and their spreading, as well as for a comparison of MRSA of different ecological origin.

Until recently the most widely used method for molecular typing of S. *aureus,* in particular MRSA, was macrorestriction pattern analysis (*Sma*I-generated). Although highly discriminatory and serially reproducible, interlaboratory comparability and portability of results are limited. This is overcome by sequence based typing which became more widely applicable with further development of sequencing techniques. Multilocus sequence typing is reflecting to core genome of bacterial pathogens and had also been introduced into population analysis of *Staphylococcus aureus* (Enright M, Robinson D, Randle G, Feil EJ, Grundmann H, Spratt BG. The evolutionary history of methicillin-resistant Staphylococcus aureus (MRSA). Proc Natl Acad Sci USA. 2002;99:7687-92). However, the necessity for sequencing at least relevant stretches of 7 house keeping genes licents the use in routine epidemiology.

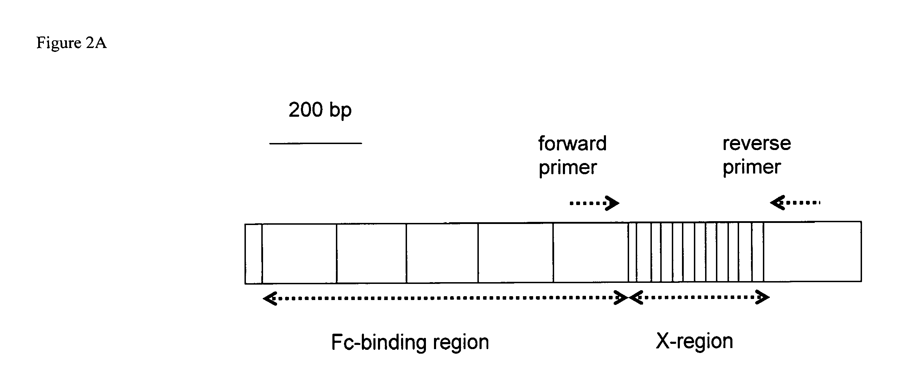

The genes for most of the S. aureus-proteins known to be attached on the cell wall contain a region of repetitive sequences. This also holds true for the so-called X-region of the gene spa (see Figure 2A). The X-region is characterised by a variable number (between 3 and 15) of small repeats. The DNA sequencing of MRSA strains furthermore revealed 25 distinct repeats, which are sufficiently stable for epidemiologic typing of MRSA strains (Frenay HM, Bunschoten AE, Schouls LM, van Leeuwen WJ, Vandenbroucke-Grauls CM, Verhoef J, Mooi FR. Molecular typing of methicillin-resistant Staphylococcus aureus on the basis of protein A gene polymorphism. Eur J Clin Microbiol Infect Dis. 1996 Jan;15(1):60-4.).

The repeats themselves furthermore contain pronounced sequence polymorphisms that appear to be based on point mutations and homologous recombinations causing inversions, deletions and duplications. By introduction of an algorithm for definition of spa-sequence types and the development of a corresponding software tool (Harmsen D, Claus H, Witte W, Rothganger J, Claus H, Turnwald D, Vogel U. Typing of methicillin-resistant Staphylococcus aureus in a university hospital setting by using novel software for spa repeat determination and database management. J Clin Microbiol. 2003 Dec;41/12):5442-8) *spa*-sequence typing became widely applicable to epidemiological studies, there is a wide congruence of *spa*-type clusters with clonal lineages as defined by MLST (Strommenger B, Kettlitz C, Weniger T, Harmsen D, Friedrich AW, Witte W. Assignment of Staphylococcus isolates to groups by spa-typing, SmaI-macrorestriction analysis, and multilocus sequence typing. J Clin Microbiol. 2006 Jul;44(7):2533-40).

Different clonal lines of MRSA can be generated by the integration of different SCC*mec-*elements into the same genome, or by the integration of identical SCC*mec*-elements into different genomes (see Figure 1). Therefore, the efficient molecular typing of MRSA must include both features of the background-genome (here the *spa*-polymorphism) as well as the SCC*mec* groups. One example for the need of such a system is the occurrence of immunity MRSA in Slovenia, it was demonstrated the different SCC*mec*-elements that these had not been generated from the widely spread nosocomial MRSA of the same sequence type (6002).

As mentioned above, different clonal lines of the species *S. aureus* show different sets of individual repeats and different arrangements of repeats. In practice, the comparison of direct sequence alignments for individual isolates is much to laborious.

It is therefore an object of the present invention, to provide a method for typing of MRSA which both allows for a sufficiently quick and reliable and at the same time cost-effective typing of MRSA, once isolates have been obtained.

According to a first aspect thereof, this object is solved by a method for typing strains or lineages of MRSA, wherein said method comprises the steps of
- providing a DNA sample comprising DNA of an MRSA strain to be analysed,
- performing a multiplex DNA amplification of said DNA in said sample, wherein at least one sequence of an SCC*mec*-element and an X-region of a *spa*-gene are co-amplified, in order to produce a master amplification-mix,
- analysing the amplified SCC*mec*-element(s) and the X-region(s) of the *spa*-gene directly from said master amplification-mix, and
- typing said strains or lineages of MRSA based on said analysis.

Other features and advantages of the present invention are described in the dependent claims, and will become readily apparent to the person of skill.

The present invention relies on a straight forward typing based on an amplification of DNA-sequences that are characteristic for each of the SCC*mec*-elements as described.

Previous multiplex PCR approaches for grouping of SCC*mec*-elements by multiplex PCR approaches have been published by Oliveira et al. (Oliveira DC, de Lencastre H. Multiplex PCR strategy for rapid identification of structural types and variants of the mec element in methicillin-resistant Staphylococcus aureus. Antimicrob Agents Chemother. 2002 Jul;46(7):2155-61.) and by Zhang et al. (Zhang K, McClure JA, Elsayed S, Louie T, Conly JM. Novel multiplex PCR assay for characterization and concomitant subtyping of staphylococcal cassette chromosome mec types I to V in methicillin-resistant Staphylococcus aureus. J Clin Microbiol 2005 Oct;43/10):5026-33). The first approach one does not include type V elements; in the second the molecular masses of the SCC*mec* specific amplimers overlap with the *spa*-amplimers; therefore both of these multiplex PCR approaches need at least one supplementary PCR for a combined amplification of SCC*mec* specific sequences and of the X-region of *spa.*

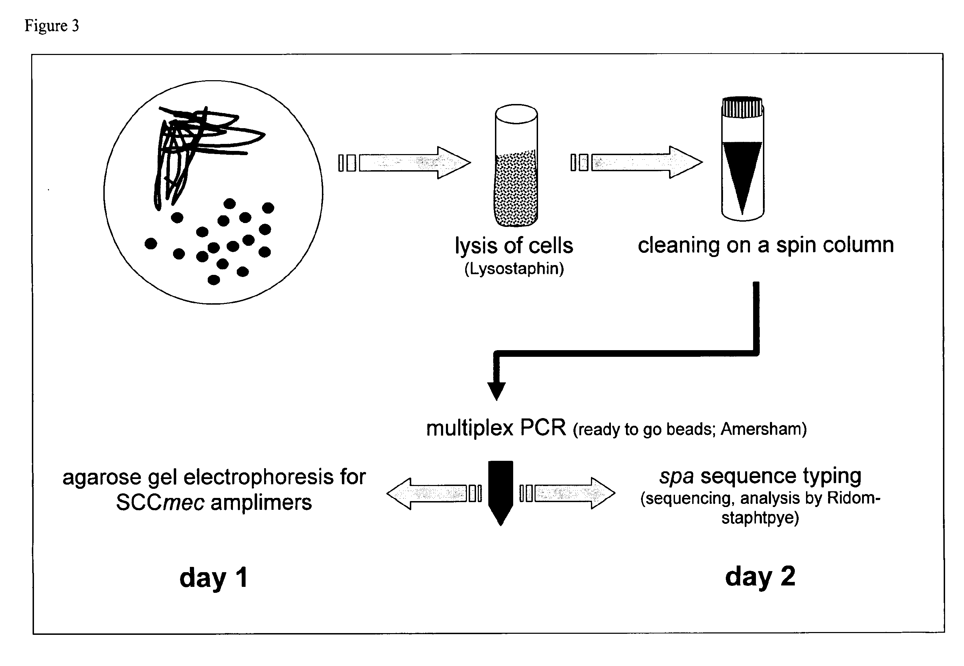

In order to overcome the above disadvantages, the present invention employs a multiplex-approach, where a separate amplification for *spa* (including the detection of the *spa*-amplimer as a control) is no longer required. This renders the analysis both faster and more cost effective, which is of particular importance in particular in a hospital environment. Figure 3 shows a schematic overview of the preferred embodiment of the method according to the present invention.

An "analysis" according to the present invention shall mean that the master amplification-mix is tested for the presence of amplification products with respect to the primers as used, and that that a portion of said master amplification-mix is directly used for a sequence analysis of at least part of the amplification product. Methods for both tests are known to the person of skill and preferred methods are further described below.

A "typing" according to the present invention shall mean that said strains or lineages of MRSA are sorted into bacterial groups based on the results of the analysis as performed in the context of the present invention. Such typing can be performed manually or, at least in part, can be supported by an apparatus, such as a robot and/or computer. Particularly preferred is the use of a software that identifies different sequences of individual repeats with, e.g., numbers, and classifies and identifies particular strains or lineages based on their order (www.ridom.com). A typing can both be made based on already known types of MRSA (preferred), nevertheless, the method according to the present invention can also be used to identify new groups of MRSA which can later be used for typing as well.

A "strain" according to the present invention is a bacterial isolate that has initially been derived from a pure culture. A strain can also be derived from a clone. A "lineage" according to the present invention is derived from a strain and includes bacteria that exhibit minor changes in their genome, when compared to the initial isolate. As an example, lineages can occur in one individual after some time due to a treatment which causes recombination and/or mutation events. Thus, for example, a patient could be infected by several lineages that have developed from one initial infection with a strain.

A preferred method according to the present invention further comprises the amplification and analysis of other elements of the SCC*mec* element(s), such as *ccr-,* transposon-, *mer*, and/or *mec*-sequences (For an example, see Figure 1). These analyses can also be performed directly from the master amplification-mix, and/or in analyses reactions that are performed parallel.

In a further preferred method according to the present invention the analysis of said amplified element(s) and/or the X-region(s) comprises a method selected from sequencing, in particular direct sequencing, gel electrophoresis, in particular agarose gel electrophoresis, restriction digestion analysis, hybridisation, and mass spectrometry. Most preferred is a method according to the present invention, wherein said analysis of the amplified *SCCmec-element(s)* comprises agarose gel electrophoresis, and of the X-region(s) of the *spa*-gene comprises direct sequencing.

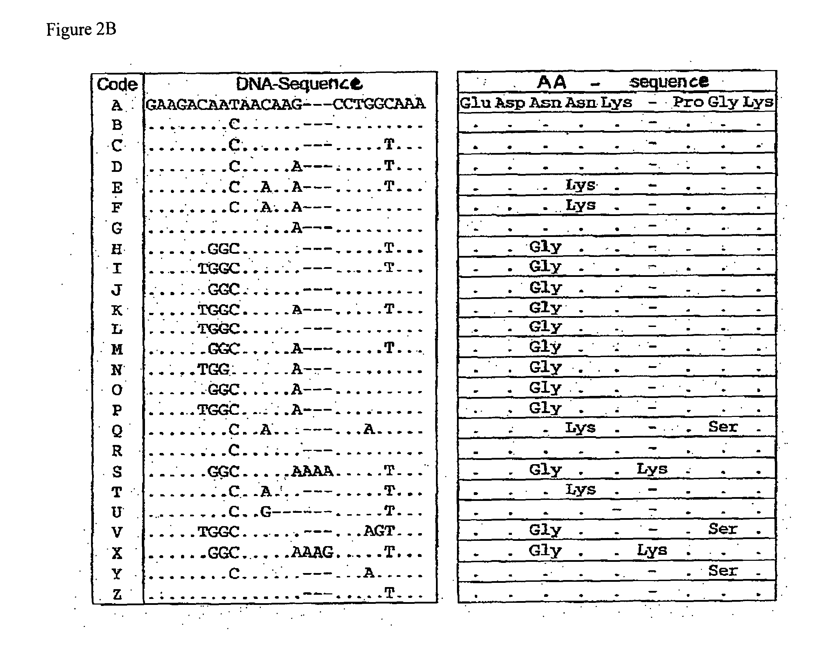

In another preferred embodiment of the method according to the present invention the analysis of the X-region(s) of the *spa-*gene comprises a determination of the number of repeats and/or a determination of the sequence of the repeats. Regarding this, reference is made to Figure 2B as an example for polymorphisms, which are determined in yet another

### preferred embodiment.

A preferred method according to the present invention further comprises an isolation of the DNA to be analysed before and/or after the amplification. Methods for preparing and/or purifying bacterial DNA are well known in the state of the art.

In another preferred method according to the present invention, the amplification comprises polymerase chain reaction, in particular rt-PCR. Nevertheless, other known DNA amplification methods can be used as well.

In yet another preferred method according to the present invention, the method further comprises a culturing of the strains and/or lines of MRSA before the analysis. This can be done in cases where a mixture of strains has been isolated and/or where the amount of DNA is still insufficient for the further analyses. Nevertheless, also several SCC*mec*-elements and/or X-regions of *spa*-genes and/or several lineages and/or hospital isolates can be analysed. In yet another preferred method according to the present invention, the sample of DNA is derived from strains and/or lineages of one individual patient or hospital.

In a most preferred embodiment of the method according to the present invention, the amplification comprises using oligonucleotides for the amplification of SCC*mec*-elements and/or the amplification of the X-regions according to any of SEQ ID No. 1 to 12. The following table shows a listing of said preferred oligonucleotides:
The discrimination of SCC*mec*-groups is based on demonstration of relevant sequences of the ccr-gene complexes; group II and IV are distinguished by PCR for the KDP-determinant in group II-elements. Primer sequences used and sizes of the amplimers are shown in table 1.

| SEQ ID No. | Sequence | Purpose | Concentration in PCR mix | amplimer size (bp) |
|---|---|---|---|---|
| 1; 24847-4868 AB033 763 | 5'- CCT.ATA.TCA.TCA.A TC.AGT.ACG.T | SCC-type I amplification forward | 0.1 µM | 180 |
| 2; 25537-5519 AB033 763 | 5’- GCC.TTG.ATA.ATA.G CC.TTC | SCC-type I amplification reverse | 0.3 µM | |
| 3; 26328-6348 D86934 | 5’- AGG.CAT.CAA.TGC.A CA.AAC.ACT | SCC-type II, IV amplification forward | 0.3 µM | 920 |
| 4; | the same as 2 | SCC-type II, IV amplification reverse | | |
| 5; 17841-7858 D86934 | 5’- GTG.CAC.GAA.CAG. AGG.AAC | SCC-type II amplification forward | 0.05 µM | 180 |
| 6; 18038-18018 D86934 | 5'- CAA.GGA.CTG.ATT.T CG.CAT.CG | SCC-type II amplification reverse | 0.05 µM | |
| 7; 5853-5873 AB047 088 | 5’-CAC- GTT.AAA.CAT.GCT.G CA.TCC | SCC-type III amplification forward | 0.1 µM | 600 |
| 8; 6469-6450 AB047 088 | 5’- TAG.CCT.TCT.TGA.G CG.CGT.CG | SCC-type III amplification reverse | 0.1 µM | |
| 9; 16421-16440 AB121 219 | 5’- ATG.AAT.TCA.AAG.A GC.ATG.GC | SCC-type V amplification forward | 0.3 µM | 120 |
| 10; 16539-16520 AB121 219 | 5’- TGA.ACG,TTC.GAT.T GA.TCC.TA | SCC-type V amplification reverse | 0.3 µM | |
| 11; 1031-1102 J01786 | 5’- TAA.AGA.CGA.TCC.T TC.AGT.GAG | Spa X-region amplification forward | 0.3 µM | 200 - 500 |
| 12; 1534-1514 J01786 | 5’- CAG.CAG.TAG.TGC.C GT.TTG-CTT | Spa X-region amplification reverse | 0.3 µM | |

In another aspect thereof, the method according to the present invention is performed, at least in part, by a suitable automate, in particular a robot. Furthermore, a suitable software can be included in the systems for performing the inventive method(s) (see above for example). The automatization leads to an additional acceleration and cost-effectiveness of the method according to the present invention.

Another aspect of the present invention then relates to a diagnostic kit, comprising materials and reagents suitable for performing a method according to the invention as above, in particular at least one oligonucleotide according to any of SEQ ID No. 1 to 12. Nevertheless, also additional components, such as a manual and or a software for analysis and typing can be provided together with said kit.

Another aspect of the present invention then relates to an improved method of treatment for an infection by MRSA, comprising a method according to the present invention, and performing a treatment of MRSA based, at least in part, on said typing of MRSA.

Finally, another aspect of the present invention then relates to a method of improving the hygiene in hospital, comprising a method according to the present invention, and performing hygiene measures, in particular additional measures, in said hospital based, at least in part, on said typing of MRSA.

Primer sequences 9 and 10 are standard primers for *spa*-sequence typing (see, for example, www.ridom.com). Primer sequences 1, 2, and 3 have been already described but slightly modified for use in this PCR. Preferred primers 3, 4, 5, 6, 7, 8 have been newly designed by the inventors.

The present invention shall now be described further in the following examples without being limited thereto. For the purposes of the present invention, all references as cited are incorporated by reference in their entireties. In the Figures and Sequences,

SEQ ID No. 1 to 12 show oligomers as used in a preferred embodiment of the present invention.

Figure 1 shows an overview of basic types of SCC*mec*-elements in MRSA.

Figure 2 shows a schematic overview about the *spa*-gene region of *S*. *aureus* (A) and the polymorphisms in the X-region thereof (B).

Figure 3 shows a diagram of the workflow of a preferred embodiment of the present invention.

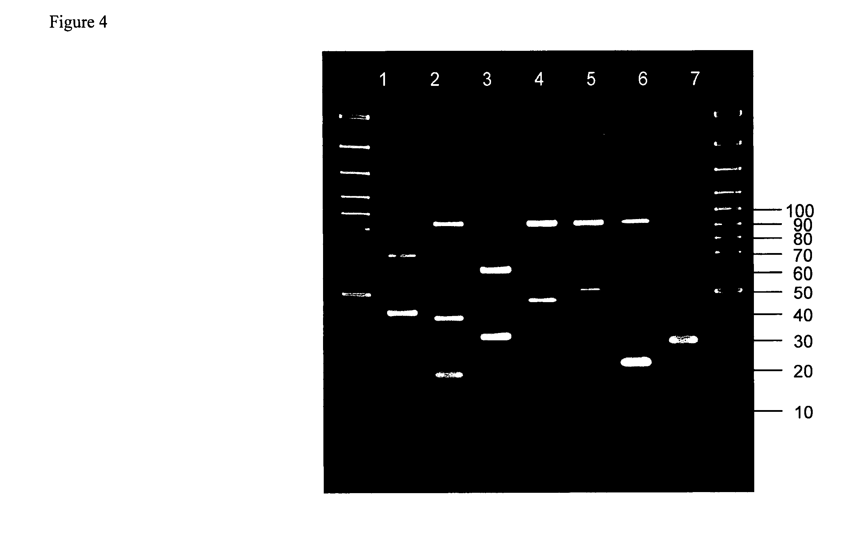

Figure 4 shows the result of a multiplex PCR according to the present invention, including controls from *mec*A and *spa.*

### Examples

### Experimental procedure for multiplex PCR:

PCR was performed by use of PuReTaq™ Ready-To-Go™ PCR beads from Amersham, Little Chalfort, Buckinghamshire, UK with 2.5 units PuReTaq™ polymerase, 10mM Tris.HCl, pH 9.0, 50m KCl, 200µM each of deoxyribonucleoside triphosphates. The concentration of MgCl₂ was adjusted to 3mM. The concentrations of primers are tabulated in table 1. After an initial denaturation step (95°C for 30 s) the amplification cycles included denaturation at 95°C for 30 s, annealing at 55°C for 30 s, and extension at 72°C for 30 s for a total of 29 cycles. The amplified products were separated on a 2 % agarose gel and visualized by ethidium bromide staining.

The experimental procedure for *spa*-sequence types has been described by Ridom StaphType, *spa*-sequencing (www.ridom.com).

In short: The sequencing reaction requires 1.0 µl premix from the kit, 1.5 µl TrisHCl/MgCl₂ buffer (400mM Tris-HCl; 10mM MgCl₂), 10 pmol of sequencing primer, and 0.25 µl from the multiplex PCR in a total volume of 10 µl. The same primers used in the PCR are used for sequencing with an annealing temperature of 60°C. All sequencing reactions are performed using a T1 Thermocycler (Whatman Biometra, Göttingen, Germany) with 25 cycles of denaturation (96°C, 10 s), and extension (60°C, 4 min). The sequencing products are purified using the Centri-Sep Spin Columns (Princeton Separations, Adelphia, NJ) and are prepared for running on the ABI 3100 Avant Genetic Analyzer in accordance with the instructions of the manufacturer (Applied Biosystems).

### Spa sequence analysis

The software Ridom StaphType is used for *spa-*sequence analysis. A comparison of separated multiplex PCR for demonstration of SCC*mec*-elements and *spa*-sequence typing to multiplex PCR including the *spa*-amplimer and which is used for sequencing revealed a complete congruence (table 2).

**Table 2: PCR for spa-sequence typing and SCCmec grouping for MRSA belonging to major clonal lineages (n = 75)**

| MLST | *spa*-type | Number of isolates checked | SCC*mec*-group (new nomenclature in brackets) | Congruence with separate PCR for *SCCmec* and *spa*-sequence typing |
|---|---|---|---|---|
| ST1 | t175 | 1 | IV (2: B1-2: B4) | 1/1 |
| ST5 | t002 | 2 | 1(1B) | 2/2 |
| ST5 | t002 | 5 | II (2A) | 5/5 |
| ST5 | t002 | 5 | V | 5/5 |
| ST8 | t008 | 4 | IV (2B) | 4/4 |
| ST8 | t008 | 2 | V (5C) | 2/2 |
| ST22 | t032 | 6 | IV (2B) | 6/6 |
| ST22 | t310 | 2 | IV (2B) | 2/2 |
| ST30 | t019 | 2 | IV (2B) | 2/2 |
| ST45 | t004 | 4 | IV (2B) | 2/2 |
| ST45 | t050 | 2 | IV (2B) | 2/2 |
| ST80 | t044 | 2 | IV (2B) | 2/2 |
| ST225 | t003 | 8 | II (2A) | 8/8 |
| ST228 | t001 | 4 | I (2A) | 4/4 |
| ST239 | t037 | 3 | III (3A) | 3/3 |
| ST247 | t051 | 4 | I (1B) | 4/4 |
| ST254 | t009 | 2 | IV (2B) | 2/2 |
| ST254 | t036 | 4 | IV (2B) | 4/4 |
| ST36 | t018 | 3 | II (2A) | 3/3 |
| ST398 | t011/t034 | 8 | V (5C) | 8/8 |
| ST398 | t034 | 2 | IV (2B) | 2/2 |

## Claims

1. Method for typing strains or lineages of MRSA, comprising the steps of
a) providing a DNA sample comprising DNA of an MRSA strain to be analysed,
b) performing a multiplex DNA amplification of said DNA in said sample, wherein at least one sequence of an SCC*mec*-element and an X-region of a *spa*-gene are co-amplified, in order to produce a master amplification-mix,
c) analysing the amplified SCC*mec*-element(s) and the X-region(s) of the *spa*-gene directly from said master amplification-mix, and
d) typing said strains or lineages of MRSA based on said analysis.

2. Method according to claim 1, further comprising the amplification and analysis of other elements of the SCC*mec* element(s), such as *ccr*-, transposon-, *mer*, and/or *mec-*sequences.

3. Method according to claim 1 or 2, wherein said analysis of said amplified element(s) and/or the X-region(s) comprises a method selected from sequencing, in particular direct sequencing, gel electrophoresis, in particular agarose gel electrophoresis, hybridisation, and mass spectrometry.

4. Method according to any of claims 1 to 3, wherein said analysis of the amplified SCC*mec*-element(s) comprises agarose gel electrophoresis, and of the X-region(s) of the spa-gene comprises direct sequencing.

5. Method according to any of claims 1 to 4, wherein said analysis of the X-region(s) of the *spa-*gene comprises a determination of the number of repeats and/or a determination of the sequence of the repeats.

6. Method according to claim 5, wherein said determination of the sequence of the repeats comprises the detection of polymorphisms.

7. Method according to any of claims 1 to 6, further comprising an isolation of the DNA to be analysed before and/or after the amplification.

8. Method according to any of claims 1 to 7, wherein said amplification comprises polymerase chain reaction, in particular rt-PCR.

9. Method according to any of claims 1 to 8, further comprising a culturing of the strains and/or lines of MRSA before the analysis.

10. Method according to any of claims 1 to 9, wherein the sample of DNA is derived from strains and/or lines of one individual patient or hospital.

11. Method according to any of claims 1 to 10, wherein several SCC*mec*-elements and/or X-regions of *spa*-genes are analysed.

12. Method according to any of claims 1 to 11, wherein several lineages and/or hospital isolates are analysed.

13. Method according to any of claims 1 to 12, wherein the amplification comprises using oligonucleotides for the amplification of SCC*mec*-elements and/or the amplification of the X-regions according to any of SEQ ID No. 1 to 12.

14. Method according to any of claims 1 to 13, wherein the method is performed, at least in part, by a suitable automate, in particular a robot.

15. Diagnostic kit, comprising materials and reagents suitable for performing a method according to any of claims 1 to 14, in particular at least one oligonucleotide according to any of SEQ ID No. 1 to 12.

16. An improved method of treatment for an infection by MRSA, comprising a method according to any of claims 1 to 14, and performing a treatment of MRSA based, at least in part, on said typing of MRSA.

17. A method of improving the hygiene in hospital, comprising a method according to any of claims 1 to 14, and performing hygiene measures in said hospital based, at least in part, on said typing of MRSA.


SEQUENCE LISTING

<110> Bundesrepublik Deutschland
vertreten durch das Bundesministerium für Gesundheit und Soziale
Sicherung letztvertreten durch den Präsidenten des Robert Koch
Institutes

<120> Supplementation of sequence based typing of methicillin-resistant
S. aureus (MRSA) by PCR-based grouping of SCCmec-elements;
sequencing directly from the multiplex-PCR

<130> FB17491

<160> 12

<170> PatentIn version 3.3

<210> 1
<211> 22
<212> DNA
<213> Staphylococcus aureus

<400> 1
cctatatcat caatcagtac gt 22

<210> 2
<211> 18
<212> DNA
<213> Staphylococcus aureus

<400> 2
gccttgataa tagccttc 18

<210> 3
<211> 21
<212> DNA
<213> Staphylococcus aureus

<400> 3
aggcatcaat gcacaaacac t 21

<210> 4
<211> 18
<212> DNA
<213> Staphylococcus aureus

<400> 4
gccttgataa tagccttc 18

<210> 5
<211> 18
<212> DNA
<213> Staphylococcus aureus

<400> 5
gtgcacgaac agaggaac 18

<210>  6
<211>  20
<212>  DNA
<213>  Staphylococcus aureus

<400>  6
caaggactga tttcgcatcg                                                    20


<210>  7
<211>  21
<212>  DNA
<213>  Staphylococcus aureus

<400>  7
cacgttaaac atgctgcatc c                                                  21


<210>  8
<211>  20
<212>  DNA
<213>  Staphylococcus aureus

<400>  8
tagccttctt gagcgcgtcg                                                    20


<210>  9
<211>  20
<212>  DNA
<213>  Staphylococcus aureus

<400>  9
atgaattcaa agagcatggc                                                    20


<210>  10
<211>  20
<212>  DNA
<213>  Staphylococcus aureus

<400>  10
tgaacgttcg attgatccta                                                    20


<210>  11
<211>  21
<212>  DNA
<213>  Staphylococcus aureus

<400>  11
taaagacgat ccttcagtga g                                                  21


<210>  12
<211>  21
<212>  DNA
<213>  Staphylococcus aureus

<400>  12
cagcagtagt gccgtttgct t                                                  21